# EUROPEAN PATENT APPLICATION

(11) **EP 4 674 408 A1**
(43) Date of publication of application: **07.01.2026**
(21) Application number: 25187323.8
(22) Date of filing: 03.07.2025
(51) Int. Cl.: A61K 9/20, A61K 31/426

(54) **A MODIFIED RELEASE TABLET COMPOSITION COMPRISING MIRABEGRON**

(30) Priority: 04.07.2024 TR 2024008630
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); KARABULUT, Serif, Istanbul (TR)
(74) Representative: Genç Ilhan, Oznur

(57) **Abstract**

The present invention relates to a modified release tablet composition comprising mirabegron and polyethylene oxide wherein; mirabegron having a d (0.9) particle size less than 50 µm and polyethylene oxide having a molecular weight of between 4,000,000 and 7,000,000.

## Description

### Field of the Invention

The present invention relates to a modified release tablet composition comprising mirabegron and polyethylene oxide wherein; mirabegron having a d (0.9) particle size less than 50 µm and polyethylene oxide having a molecular weight of between 4,000,000 and 7,000,000.

### Background of the Invention

Overactive bladder causes a frequent and sudden urge to urinate that may be difficult to control. You may feel like you need to pass urine many times during the day and night, and may also experience unintentional loss of urine.

There are many options for treatment of overactive bladder such as; bladder training and drug therapy using anticholinergic substances such as propiverine hydrochloride and oxybutynin hydrochloride have been mostly used at present. However, intractable cases and side effects could be occurred such as urinary dysfunction and dry mouth and, therefore, Mirabegron has been reported as one of the substance for the management of overactive bladder.

Mirabegron is a beta-3 adrenergic agonist that is used for treatment of overactive bladder syndrome. The chemical designation of mirabegron is 2-(2-amino-1,3-thiazol-4-yl)-N-[4-[2-[[(2R)-2-hydroxy-2-phenylethyl]amino]ethyl]phenyl] acetamide, with the chemical structure illustrated below in Formula I.

### Formula I

A mirabegron containing pharmaceutical product is approved under the brand name Betmiga^{®} in the EU and Mirbetriq^{®} in the US as modified release tablets comprising 25 and 50 mg of mirabegron.

Mirabegron is considered to be a Class III compound according to the Biopharmaceutical Classification System (BCS). That means that it has high solubility and low permeability. It is known that the bioavailability of mirabegron is affected by the presence of food in the GI tract. To prevent this food effect, modified release form is used.

EP1559427 patent application was first disclosed a pharmaceutical composition comprising mirabegron that can be used as a therapeutic agent for overactive bladder, such as overactive bladder accompanied by prostatic hyperplasia, or overactive bladder accompanied by urinary urgency, urinary incontinence, and urinary frequency.

WO2018169325 relates to a controlled release pharmaceutical composition comprising mirabegron or a pharmaceutically acceptable salt thereof and a polyethylene oxide as a sustained release agent.

In prior art, there are also several patents which disclose a composition comprising mirabegron. However, despite the dissolution profile and stability problems of compositions comprising mirabegron, an effective formulation and method has not been disclosed.

There still remains a need in the art to provide an improved modified release tablet composition of mirabegron, having high solubility, dissolution rate, and excellent pharmacotechnical properties such as flowability, compressibility and homogeneity.

### Detailed Description of the Invention

The main object of the present invention is to provide a modified release tablet composition comprising mirabegron with having desired level of dissolution rate which overcomes the above-described problems in prior art and have additive advantages over them.

Another object of the present invention is to provide a modified release tablet composition comprising mirabegron with improved homogeneity, flowability and high stability.

Another object of the present invention is to provide a modified release tablet composition comprising mirabegron prepared by simple, easy, time-saving and fast manufacturing methods.

The term "modified release" refers to any pharmaceutical formulation that maintain constant levels of a drug in the patient's bloodstream by releasing the drug over an extended period of time. Modified release is formulated to release the active ingredient gradually and predictably over a 12-hour to 24-hour period. In this invention, using release controlling agent (polyethylene oxide) provides modified release.

According to an embodiment of the present invention, the release controlling agent is polyethylene oxide. Polyethylene oxide is a very hydrophilic polymer. PEO polymers are available in a range of molecular weights used to tailor the drug release for various dose/solubility characteristics of the drug. For any particular drug in a formulation, substituting PEO grades by increasing molecular weight grades, while maintaining a constant polymer concentration can significantly reduce release rates.

In this invention, the high viscosity grade of PEOs are used. These PEO polymers are polyethylene oxide (PEO) having a molecular weight of between 4,000,000 and 7,000,000. Used polyethylene oxide can be polyethylene oxide WSR - 303 NF, polyethylene oxide WSR Coagulant NF, polyethylene oxide WSR - 301 NF.

As used here in, 'particle size' means the cumulative volume size distribution as tested by any conventionally accepted method such as the laser diffraction method (i.e. Malvern analysis). The term d (0.9) means, the size at which 90% by volume of the particles are finer.

According to main embodiment of the present invention, the modified release tablet composition comprising mirabegron and polyethylene oxide wherein; mirabegron having a d (0.9) particle size less than 50 µm and polyethylene oxide having a molecular weight of between 4,000,000 and 7,000,000.

The inventor surprisingly discovered that when mirabegron particle size is larger than 50 µm, it is prone to sticking during tablet production, resulting in defects in the surface shape of the tablets.

The use of mirabegron in the specified particle sizes ensured content uniformity and ensured homogeneity in the mixture. This helped to achieve the desired dissolution profile. Also, there is no problems with the tablet surface.

According to this embodiment of the present invention, mirabegron has a d (0.9) particle size between 1 µm and 50 µm, preferably between 7 µm and 38 µm, more preferably between 15 µm and 40 µm.

In the invention, polyethylene oxide has an average molecular weight of approximately 7,000,000 with a viscosity of 7500 to 10000 cps at a 1% aqueous solution at 25° C.

The modified tablet composition of the present invention comprises a core tablet and a film coating. The weight of the film coating is included in all calculations and the calculations are expressed as a percentage by weight of the total composition.

According to an embodiment of the present invention, the amount of polyethylene oxide is 10.0% to 30.0% by weight in the total composition. Preferably, it is 14.0% to 20.0% by weight in the total composition.

According to an embodiment of the present invention, the amount of mirabegron is 15.0% to 40.0% by weight in the total composition. Preferably, it is between 15.0% to 32.0% by weight in the total composition. More preferably, it is between 18.0% to 25.0% by weight in the total composition.

According to an embodiment of the present invention, the modified release tablet composition comprises;
- 15.0% and 40.0% by weight of Mirabegron having a d (0.9) particle size less than 50 µm,
- 10.0% and 30.0% by weight of Polyethylene oxide having a molecular weight of between 4,000,000 and 7,000,000.

According to one embodiment of the present invention, the modified release tablet composition further comprises at least one pharmaceutically acceptable excipient which is selected from the group comprising diluents, binders, antioxidants, lubricants or mixtures thereof.

Suitable diluents are selected from the group comprising polyethylene glycol, microcrystalline cellulose, lactose monohydrate, ammonium alginate, calcium carbonate, calcium phosphate, calcium phosphate dehydrate, neutral pellets, calcium sulfate, cellulose, cellulose acetate, lactose, mannitol, magnesium carbonate, medium chain triglycerides, polyvinylpyrrolidone, sodium alginate, sodium chloride, sucrose, sugar spheres, or mixtures thereof.

According to an embodiment of the present invention, the diluent is polyethylene glycol. It gives volume to tablet and enables mirabegron to be dispersed homogeneously in the tablet.

According to an embodiment of the present invention, the amount of polyethylene glycol is 1.0% to 10.0% by weight in the total composition. Preferably, it is 2.0% to 4.5% by weight in the total composition.

Suitable binders are selected from the group comprising hydroxypropyl cellulose, sodium carboxymethyl cellulose, polyvinylpyrrolidone (K30, K90), sodium carboxymethyl cellulose, starch, pregelatinized starch, sodium alginate, hydroxypropyl methyl cellulose, carboxy methyl cellulose, methyl cellulose, gelatin, carrageenan, guar gum, polymethacrylates, methacrylate polymers, polysaccharides, carbomer, poloxamer, polydextrose or mixtures thereof.

According to an embodiment of the present invention, the binder is hydroxypropyl cellulose.

According to an embodiment of the present invention, the amount of hydroxypropyl cellulose is 1.0% to 10.0% by weight in the total composition. Preferably, it is 2.0% to 5.0% by weight in the total composition.

Suitable antioxidants are selected from the group comprising BHT (butylated hydroxy toluene), propyl gallate, BHA (butylated hydroxy anisole), monothioglycerol or mixtures thereof.

According to an embodiment of the present invention, the antioxidant is BHT (butylated hydroxy toluene). PEO having the high viscosity grade is more prone to viscosity loss compared to PEO having lower viscosity grade. Hence, PEO polymers having the high molecular weight are stabilized by addition of butylated hydroxy toluene (BHT).

According to an embodiment of the present invention, the amount of butylated hydroxytoluene is 0.05% to 2.0% by weight in the total composition. Preferably, it is 0.10% to 1.0% by weight in the total composition.

Suitable lubricant is selected from the group comprising magnesium stearate, polyoxyl 40 stearate, calcium stearate, sodium stearyl fumarate, potassium stearate, stearic acid, high melting point waxes, sodium chloride, sodium benzoate, sodium acetate, sodium oleate or mixtures thereof.

According to an embodiment of the present invention, the lubricant is magnesium stearate. It enhances product flow by reducing inter particulate friction.

According to an embodiment of the present invention, the amount of magnesium stearate is 0.05% to 2.0% by weight in the total composition.

According to an embodiment of the present invention, the modified release tablet composition comprises;
- Mirabegron having a d (0.9) particle size less than 50 µm,
- Polyethylene oxide having a molecular weight of between 4,000,000 and 7,000,000
- PEG 8000
- Hydroxypropyl Cellulose
- Butylated Hydroxytoluene
- Magnesium stearate

Furthermore, the modified release tablet composition is obtained by using dry granulation or direct compression and therefore a simple and low-cost production method was employed. This process provides drug compatibility and content uniformity.

### Example 1: Film coated tablet having modified release

| **Ingredients** | **Amount (% by weight of the total composition)** |
|---|---|
| Mirabegron having a d (0.9) particle size less than 50 µm | 15.0 - 40.0 |
| Polyethylene oxide having a molecular weight of between 4,000,000 and 7,000,000 | 10.0 - 30.0 |
| PEG 8000 | 40.0 - 65.0 |
| Hydroxypropyl Cellulose | 1.0 - 10.0 |
| Butylated Hydroxytoluen | 0.05 - 2.0 |
| Magnesium stearate | 0.05 - 2.0 |
| **TOTAL** | **100** |
| **Film Coating** | |

### Example 2: Film coated tablet having modified release

| **Ingredients** | **Amount (% by weight of the total composition)** |
|---|---|
| Mirabegron having a d (0.9) particle size less than 50 µm | 20.00 |
| Polyethylene oxide having a molecular weight of between 4,000,000 and 7,000,000 | 16.50 |
| PEG 8000 | 59.34 |
| Hydroxypropyl Cellulose (HPC) | 3.00 |
| Butylated Hydroxytoluen | 0.16 |
| Magnesium stearate | 1.00 |
| **TOTAL** | **100** |
| **Film Coating** | |

A process for example 1 or 2 ;
- Sieving mirabegron, Hydroxypropyl Cellulose, polyethylene glycol, polyethylene oxide into 0.9 mm,
- Adding butylated hydroxytoluen and then mixing,
- Adding the half of magnesium stearate and then mixing,
- Performing compaction with the mixture
- Sieving the mixture into 1.5 mm and then mixing,
- Adding the remaining of magnesium stearate and then mixing,
- Compressing the mixture into the tablet,
- Coating the tablets with film coating agent.

### Film coating

| |
|---|
| HPMC |
| IRON OXIDE YELLOW |
| GLYCERIN |

## Claims

1. A modified release tablet composition comprising mirabegron and polyethylene oxide wherein; mirabegron having a d (0.9) particle size less than 50 µm and polyethylene oxide having a molecular weight of between 4,000,000 and 7,000,000.

2. The modified release tablet composition according to claim 1, wherein mirabegron has a d (0.9) particle size between 1 µm and 50 µm, preferably between 7 µm and 38 µm, more preferably between 15 µm and 40 µm.

3. The modified release tablet composition according to claim 1, wherein the amount of polyethylene oxide is 10.0% to 30.0% by weight in the total composition.

4. The modified release tablet composition according to claim 1, wherein the amount of mirabegron is 15.0% to 40.0% by weight in the total composition.

5. The modified release tablet composition according to claim 3 or 4, wherein the tablet comprising;
- 15.0% and 40.0% by weight of Mirabegron having a d (0.9) particle size less than 50 µm,
- 10.0% and 30.0% by weight of Polyethylene oxide having a molecular weight of between 4,000,000 and 7,000,000.

6. The modified release tablet composition according to any preceding claims, wherein the tablet further comprising at least one pharmaceutically acceptable excipient which is selected from the group comprising diluents, binders, antioxidants, lubricants or mixtures thereof.

7. The modified release tablet composition according to claim 6, wherein diluents are selected from the group comprising polyethylene glycol, microcrystalline cellulose, lactose monohydrate, ammonium alginate, calcium carbonate, calcium phosphate, calcium phosphate dehydrate, neutral pellets, calcium sulfate, cellulose, cellulose acetate, lactose, mannitol, magnesium carbonate, medium chain triglycerides, polyvinylpyrrolidone, sodium alginate, sodium chloride, sucrose, sugar spheres, or mixtures thereof.

8. The modified release tablet composition according to claim 6 or 7, wherein the diluent is polyethylene glycol.

9. The modified release tablet composition according to claim 8, wherein the amount of polyethylene glycol is 1.0% to 10.0% by weight in the total composition.

10. The modified release tablet composition according to claim 8, wherein the amount of polyethylene glycol is 2.0% to 4.5% by weight in the total composition.

11. The modified release tablet composition according to claim 6, wherein binders are selected from the group comprising hydroxypropyl cellulose, sodium carboxymethyl cellulose, polyvinylpyrrolidone (K30, K90), sodium carboxymethyl cellulose, starch, pregelatinized starch, sodium alginate, hydroxypropyl methyl cellulose, carboxy methyl cellulose, methyl cellulose, gelatin, carrageenan, guar gum, polymethacrylates, methacrylate polymers, polysaccharides, carbomer, poloxamer, polydextrose or mixtures thereof.

12. The modified release tablet composition according to claim 11, wherein the binder is hydroxypropyl cellulose.

13. The modified release tablet composition according to claim 6, wherein antioxidants are selected from the group comprising BHT (butylated hydroxy toluene), propyl gallate, BHA (butylated hydroxy anisole), monothioglycerol or mixtures thereof.

14. The modified release tablet composition according to claim 6 or 13, wherein the antioxidant is BHT (butylated hydroxy toluene).

15. The modified release tablet composition according to claim 6, wherein the tablet comprising;
- Mirabegron having a d (0.9) particle size less than 50 µm,
- Polyethylene oxide having a molecular weight of between 4,000,000 and 7,000,000
- Hydroxypropyl Cellulose
- Butylated Hydroxytoluene
- Magnesium stearate
